(19) Europäisches Patentamt
European Patent Office
Office européen des brevets

(11) **EP 2 932 890 A1**

(12) **EUROPÄISCHE PATENTANMELDUNG**

(43) Veröffentlichungstag:
**21.10.2015 Patentblatt 2015/43**

(21) Anmeldenummer: **15162800.5**

(22) Anmeldetag: **08.04.2015**

(51) Int Cl.:
*A61B 5/00* *(2006.01)*          *A61B 5/055* *(2006.01)*
*G01R 33/563* *(2006.01)*      *A61B 8/00* *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **17.04.2014 DE 102014207513**

(71) Anmelder:
• **Charité - Universitätsmedizin Berlin**
  **10117 Berlin (DE)**
• **Cermakova, Iva**
  **09112 Chemnitz (DE)**

(72) Erfinder:
• **Braun, Jürgen**
  **14169 Berlin (DE)**
• **Sack, Ingolf**
  **10437 Berlin (DE)**
• **Hirsch, Sebastian**
  **13409 Berlin (DE)**
• **Cermak, Ivo**
  **09112 Chemnitz (DE)**

(74) Vertreter: **Fischer, Uwe**
**Patentanwalt**
**Moritzstraße 22**
**13597 Berlin (DE)**

(54) **ELASTOGRAPHIEEINRICHTUNG UND VERFAHREN ZU DEREN BETRIEB**

(57) Eine Elastographieeinrichtung (10) wird betrieben, indem mit einer Anregungseinheit (20) mechanische Schwingungsmuster zur Erzeugung mechanischer Gewebebewegungen in einem menschlichen oder tierischen Gewebe erzeugt werden und mit einer Bildaufnahmeeinrichtung (30) während der Gewebebewegungen Bilder des Gewebes erzeugt werden.

Zur Auslösung des jeweils nächsten mechanischen Schwingungsmusters wird in einen Triggersignalanschluss (20a) der Anregungseinheit (20) jeweils ein Triggersignal (T) eingespeist. Zur Konfiguration der Anregungseinheit (20) werden Konfigurationssignale (K) in denselben Triggersignalanschluss (20a) der Anregungseinheit (20) eingespeist.

Fig.3

**Beschreibung**

Die Erfindung bezieht sich auf ein Verfahren zum Betreiben einer Elastographieeinrichtung, bei dem mit einer Anregungseinheit mechanische Schwingungsmuster zur Erzeugung mechanischer Gewebebewegungen in einem menschlichen oder tierischen Gewebe erzeugt werden und mit einer Bildaufnahmeeinrichtung während der Gewebebewegungen Bilder des Gewebes erzeugt werden.

Bekanntermaßen erlauben moderne medizinische Bildaufnahmeeinrichtungen, nachfolgend auch Bildgebungsmodalitäten genannt, - wie zum Beispiel die Magnetresonanztomographie (MRT) oder die Sonographie - neben der Darstellung von morphologischem Weichgewebekontrast einzigartige Möglichkeiten zur Bestimmung von Bewegung, Fluss und Funktion im lebenden Organismus. Eine wichtige Anwendung der MRT und der Sonographie ist die ortsaufgelöste Darstellung viskoelastischer Kenngrößen weicher Gewebe, die Elastographie, welche die sensitive bildgestützte Palpation zur Erkennung von Krankheiten erlaubt. Ein wesentliches Anwendungsgebiet der Elastographie mittels MRT und Ultraschall ist die nichtinvasive Graduierung der Leberfibrose [1,2]. Ein technisches Hauptmerkmal der Elastographie stellt die externe mechanische Stimulation des zu untersuchenden Gewebes mittels einer Anregungseinheit dar. Eine Anregungseinheit umfasst üblicherweise einen Signalgenerator (bzw. Signalformgenerator), einen Verstärker und einen geeigneten Bewegungswandler, der die mechanische Deformation des zu untersuchenden Gewebes mit der gewünschten Bewegungstrajektorie ausführt [3]. Die externe Stimulation sollte möglichst synchron zur Bildaufnahme erfolgen, um durch die zeitliche Kontrolle der Deformationsmuster Aufschluss über die zugrunde liegenden mechanischen Eigenschaften des Gewebes zu erlangen. Aus der Notwendigkeit der Synchronisation der Anregungseinheit mit der Bildaufnahmeeinrichtung ergibt sich die Problemstellung einer Kommunikation zwischen diesen Geräten mit minimaler Latenz bzw. Verzögerung.

Der Erfindung liegt die Aufgabe zugrunde, ein Verfahren zum Betreiben einer Elastographieeinrichtung anzugeben, bei dem eine möglichst verzögerungsarme Kommunikation zwischen der Bildaufnahmeeinrichtung und der Anregungseinheit insbesondere mit Blick auf eine etwaige Änderung der Betriebsparameter während einer Messung erreicht wird.

Diese Aufgabe wird erfindungsgemäß durch ein Verfahren mit den Merkmalen gemäß Patentanspruch 1 gelöst. Vorteilhafte Ausgestaltungen des erfindungsgemäßen Verfahrens sind in Unteransprüchen angegeben.

Danach ist erfindungsgemäß vorgesehen, dass zur Auslösung des jeweils nächsten mechanischen Schwingungsmusters in einen Triggersignalanschluss der Anregungseinheit jeweils ein Triggersignal eingespeist wird und dass zur Konfiguration der Anregungseinheit Konfigurationssignale in denselben Triggersignalanschluss der Anregungseinheit eingespeist werden.

Ein wesentlicher Vorteil des erfindungsgemäßen Verfahrens besteht darin, dass zur Triggerung und Konfiguration der Anregungseinheit ein und dieselbe Signalleitung herangezogen wird. Da Signalleitungen, die zur Übertragung von Triggersignalen bestimmt sind, also Triggersignalleitungen, per se eine latenzarme (verzögerungsarme) bzw. latenzfreie (verzögerungsfreie) Signalübertragung gewährleisten müssen, wird allein durch die erfindungsgemäße Doppelnutzung der Triggersignalleitung eine latenzarme bzw. latenzfreie Signalübertragung auch für die Konfigurationssignale gewährleistet, und dies in vorteilhafter Weise ohne zusätzliche Hardwarekosten.

Als besonders vorteilhaft wird es angesehen, wenn die Anregungseinheit am Triggersignalanschluss anliegende Signale mit einer Auswerteinrichtung auswertet und Triggersignale zur Auslösung des jeweils nächsten Schwingungsmusters von Konfigurationssignalen unterscheidet. Im Falle des Eingangs eines Konfigurationssignals wird die in dem Konfigurationssignal definierte Konfigurierung von der Anregungseinheit vorgenommen.

Die Auswerteinrichtung leitet vorzugsweise an dem Triggersignalanschluss der Anregungseinheit anliegende Triggersignale zur Auslösung des jeweils nächsten Schwingungsmusters an den Triggereingang eines Signalgenerators der Anregungseinheit weiter. An dem Triggersignalanschluss der Anregungseinheit anliegende Konfigurationssignale, die jeweils ein in der Anregungseinheit, insbesondere in dem Signalgenerator, abgespeichertes Signalmuster aus einer Gruppe von in der Anregungseinheit, insbesondere in dem Signalgenerator, abgespeicherten Signalmustern auswählen bzw. definieren, leitet die Auswerteinrichtung vorzugsweise an einen Auswahleingang des Signalgenerators zur Auswahl des jeweiligen Signalmusters weiter.

Bei den Signalmustern handelt es sich vorzugsweise um harmonische Signalmuster, also sinusförmige Signalmuster mit einer individuell zugeordneten (einzigen) Schwingfrequenz, einer individuell zugeordneten Amplitude und einer individuell zugeordneten zeitlichen Signalmusterlänge. Alternativ können die Signalmuster auch durch eine Überlagerung bzw. Superposition zweier oder mehrerer harmonischer bzw. sinusförmiger Schwingfrequenzen gebildet sein.

Auch kann vorgesehen werden, dass die Auswerteinrichtung an dem Triggersignalanschluss der Anregungseinheit anliegende Konfigurationssignale, die jeweils eine zeitliche Synchronisation einer internen Zeitbasis (Uhr) der Bildaufnahmeeinrichtung mit einer internen Zeitbasis (Uhr) der Anregungseinheit hervorrufen sollen, an die interne Zeitbasis der Anregungseinheit weiterleitet. Alternativ kann für eine Synchronisierung der beiden internen Zeitbasen eine separate Synchronisationsleitung zwischen der Bildaufnahmeeinrichtung und der Anregungseinheit eingesetzt werden.

Die Unterscheidung eines Triggersignals von einem Konfigurationssignal erfolgt vorzugsweise anhand der Pulslänge und/- oder der Pulsamplitude des Signals oder zumindest eines ersten Pulses des jeweiligen Signals.

Die Auswerteinrichtung gibt vorzugsweise Statussignale, die den jeweiligen Gerätestatus der Anregungseinheit angeben, an dem Triggersignalanschluss der Anregungseinheit aus und überträgt diese über den Triggersignalanschluss an den Signalausgang der Bildaufnahmeeinrichtung. Bei dieser Ausgestaltung erfolgt also eine bidirektionale Signalübertragung zwischen dem Triggersignalanschluss der Anregungseinheit und dem Signalausgang der Bildaufnahmeeinrichtung; um eine Datenkollision zu vermeiden, arbeitet die Anregungseinheit vorzugsweise als Slave und die Bildaufnahmeeinrichtung vorzugsweise als Master. Beispielsweise kann die Bildaufnahmeeinrichtung Daten von der Anregungseinheit durch einen Puls oder eine Pulssequenz anfordern und auf die Daten warten, ohne in der Zeit selbst etwas zu senden.

Die Erfindung bezieht sich außerdem auf eine Elastographieeinrichtung, die ausgestattet ist mit einer Anregungseinheit zur Erzeugung mechanischer Schwingungsmuster und zur Erzeugung mechanischer Gewebebewegungen in einem menschlichen oder tierischen Gewebe und einer Bildaufnahmeeinrichtung. Erfindungsgemäß ist vorgesehen, dass die Bildaufnahmeeinrichtung einen Signalausgang aufweist, den sie wahlweise als Triggersignal- oder Konfigurationssignalausgang betreiben kann und über den sie wahlweise Triggersignale zur Auslösung eines mechanischen Schwingungsmusters oder Konfigurationssignale in einen Triggersignalanschluss der Anregungseinheit einspeisen kann. Die Anregungseinheit weist eine mit dem Triggersignalanschluss in Verbindung stehende Auswerteinrichtung auf, die derart ausgestaltet ist, dass sie am Triggersignalanschluss anliegende Signale auswertet und die Triggersignale zur Auslösung eines mechanischen Schwingungsmusters von den Konfigurationssignalen unterscheidet.

Bezüglich der Vorteile der erfindungsgemäßen Elastographieeinrichtung sei auf die obigen Ausführungen im Zusammenhang mit dem erfindungsgemäßen Verfahren verwiesen, da die Vorteile des Verfahrens für die erfindungsgemäße Elastographieeinrichtung entsprechend gelten.

Die Anregungseinheit weist vorzugsweise einen Signalgenerator und einen mit dem Signalgenerator verbundenen elektromechanischen Wandler auf, der elektrische Signalmuster des Signalgenerators in korrespondierende mechanische Schwingungsmuster umsetzt. Ein Triggereingang des Signalgenerators steht vorzugsweise mit einem Triggersignalausgang der Auswerteinrichtung in Verbindung.

Die Auswerteinrichtung ist vorzugsweise derart ausgestaltet, dass sie an dem Triggersignalanschluss der Anregungseinheit anliegende Triggersignale zur Auslösung eines mechanischen Schwingungsmusters an dem Triggersignalsausgang der Auswerteinrichtung ausgibt und über diesen in den Triggereingang des Signalgenerators einspeist.

Außerdem ist die Auswerteinrichtung vorzugsweise derart ausgestaltet, dass sie an dem Triggersignalanschluss der Anregungseinheit anliegende Konfigurationssignale, die jeweils ein in dem Signalgenerator abgespeichertes Signalmuster definieren, über einen Konfigurationsausgang an einen Auswahleingang des Signalgenerators zur Auswahl des jeweiligen Signalmusters weiterleitet.

Auch kann vorgesehen sein, dass die Auswerteinrichtung an dem Triggersignalanschluss anliegende Konfigurationssignale, die jeweils eine zeitliche Synchronisation einer internen Zeitbasis (Uhr) der Bildaufnahmeeinrichtung mit einer internen Zeitbasis (Uhr) der Anregungseinheit hervorrufen sollen, an die interne Zeitbasis der Anregungseinheit weiterleitet. Alternativ kann zur Synchronisation der Zeitbasen auch eine zusätzliche Synchronisationsleitung vorhanden sein.

Als besonders vorteilhaft wird es angesehen, wenn die Auswerteinrichtung derart ausgestaltet ist, dass sie im Falle eines am Triggersignalanschluss anliegenden Signals

- die Pulslänge und/oder die Pulsamplitude des jeweiligen Signals oder
- die Pulslänge und/oder die Pulsamplitude eines ersten Pulses (Einzelpulses) des jeweiligen Signals

auswertet und die Feststellung, ob es sich um ein Triggersignal zur Auslösung eines mechanischen Schwingungsmusters oder um ein Konfigurationssignal handelt, anhand der Pulslänge und/oder der Pulsamplitude trifft.

Die Erfindung bezieht sich außerdem auf eine Anregungseinheit für eine Elastographieeinrichtung, insbesondere eine solche, wie sie oben beschrieben worden ist. Erfindungsgemäß ist bezüglich einer solchen Anregungseinheit vorgesehen, dass diese eine mit einem Triggersignalanschluss der Anregungseinheit in Verbindung stehende Auswerteinrichtung aufweist, die derart ausgestaltet ist, dass sie am Triggersignalanschluss anliegende Signale auswertet und Triggersignale zur Auslösung eines mechanischen Schwingungsmusters von Konfigurationssignalen zur Konfigurierung der Anregungseinheit trennt.

Bezüglich der Vorteile der erfindungsgemäßen Anregungseinheit sei auf die obigen Ausführungen im Zusammenhang mit der erfindungsgemäßen Elastographieeinrichtung verwiesen, da die Vorteile der erfindungsgemäßen Elastographieeinrichtung denen der erfindungsgemäßen Anregungseinheit entsprechen.

Die Erfindung bezieht sich außerdem auf eine Bildaufnahmeeinrichtung für eine Elastographieeinrichtung, insbesondere eine solche, wie sie oben beschrieben worden ist. Erfindungsgemäß ist bezüglich einer solchen Bildaufnahmeeinrichtung vorgesehen, dass diese einen Signalausgang aufweist, den sie wahlweise als Triggersignal- oder Konfigurationssignalausgang betreiben kann und über den sie wahlweise Triggersignale zur Auslösung eines mechanischen Schwingungsmusters oder Konfigurationssignale zur Konfiguration einer Anregungseinheit in einen Triggersignalanschluss der Anregungseinheit einspeisen kann.

Bezüglich der Vorteile der erfindungsgemäßen Bildaufnahmeeinrichtung sei auf die obigen Ausführungen im Zusammenhang mit der erfindungsgemäßen Elastographieeinrichtung verwiesen, da die Vorteile der erfindungsgemäßen Elastographieeinrichtung denen der erfindungsgemäßen Bildaufnahmeeinrichtung entsprechen.

Die Erfindung wird nachfolgend anhand von Ausführungsbeispielen näher erläutert; dabei zeigen beispielhaft:

Figur 1    ein erstes Ausführungsbeispiel für eine erfindungsgemäße Elastographieeinrichtung, anhand derer beispielhaft auch eine Ausführungsvariante des erfindungsgemäßen Verfahrens erläutert wird,

Figur 2    ein zweites Ausführungsbeispiel für eine erfindungsgemäße Elastographieeinrichtung, anhand derer beispielhaft eine andere Ausführungsvariante des erfindungsgemäßen Verfahrens erläutert wird, und

Figur 3    beispielhaft den Zeitverlauf von Steuerpulsen P am Triggersignalanschluss einer Auswerteinheit der Elastographieeinrichtung gemäß den Figuren 1 und 2 sowie den zeitlichen Verlauf der von einem Signalgenerator der Elastographieeinrichtung zur Ansteuerung eines elektromechanischen Wandlers ausgegebenen elektrischen Signalmuster.

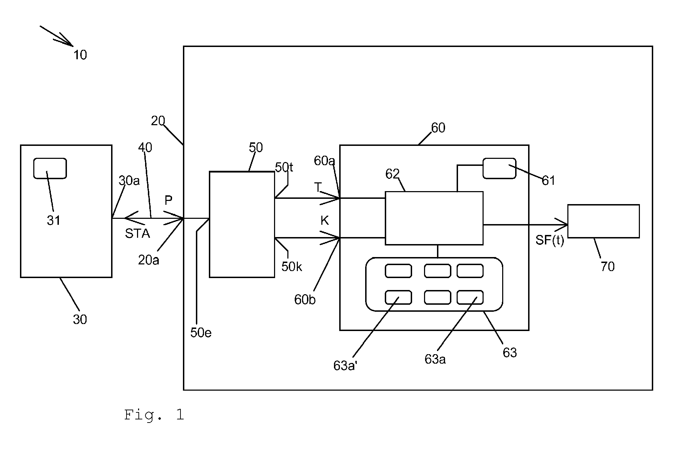

Die Figur 1 zeigt ein Ausführungsbeispiel für eine Elastographieeinrichtung 10. Die Elastographieeinrichtung 10 umfasst eine Anregungseinheit 20 zur Erzeugung mechanischer Schwingungsmuster und zur Erzeugung mechanischer Gewebebewegungen in einem menschlichen oder tierischen Gewebe. Darüber hinaus umfasst die Elastographieeinrichtung 10 eine Bildaufnahmeeinrichtung 30.

Ein Signalausgang 30a der Bildaufnahmeeinrichtung 30 steht über eine Signalleitung 40 mit einem Triggersignalanschluss 20a der Anregungseinheit 20 in Verbindung. Über diesen Triggersignalanschluss 20a und damit über die Signalleitung 40 lassen sich Triggersignale T zur Auslösung der Anregungseinheit 20 und zur Erzeugung des jeweils nächsten mechanischen Schwingungsmusters einspeisen. Darüber hinaus lassen sich über den Triggersignalanschluss 20a und die Signalleitung 40 Konfigurationssignale K zur Konfiguration der Anregungseinheit 20 übertragen.

Die Signalleitung 40 kann eine elektrische oder optische Leitung sein; alternativ kann anstelle der Signalleitung 40 eine drahtlose Verbindung (z. B Funkverbindung) als Kommunikationsverbindung bzw. Kommunikationskanal eingesetzt werden.

Die Anregungseinheit 20 weist eine mit dem Triggersignalanschluss 20a in Verbindung stehende Auswerteinrichtung 50 auf, die derart ausgestaltet ist, dass sie am Triggersignalanschluss 20a anliegende Signale auswertet und Triggersignale T zur Auslösung mechanischer Schwingungsmuster von Konfigurationssignalen K unterscheidet.

Die Anregungseinheit 20 umfasst außerdem einen Signalgenerator 60 und einen mit dem Signalgenerator 60 verbundenen elektromechanischen Wandler 70, der elektrische Signalmuster des Signalgenerators 60 in korrespondierende mechanische Schwingungsmuster umsetzt.

Ein Triggereingang 60a des Signalgenerators 60 steht mit einem Triggersignalausgang 50t der Auswerteinrichtung 50 in Verbindung, so dass die Auswerteinrichtung 50 an dem Triggersignalanschluss 20a der Anregungseinheit 20 anliegende Triggersignale T zur Auslösung des jeweils nächsten mechanischen Schwingungsmusters über den Triggersignalausgang 50t an den Triggereingang 60a des Signalgenerators 60 weiterleiten kann.

Die Auswerteinrichtung 50 trennt an dem Triggersignalanschluss 20a der Anregungseinheit 20 anliegende Triggersignale T von dort anliegenden Konfigurationssignalen K, die beispielsweise ein in dem Signalgenerator 60 abgespeichertes Signalmuster auswählen bzw. definieren, und leitet diese über einen Konfigurationsausgang 50k an einen Auswahleingang 60b des Signalgenerators 60 weiter.

Bei dem Ausführungsbeispiel gemäß Figur 1 kann die Auswerteinrichtung 50 außerdem Konfigurationssignale K, die jeweils eine zeitliche Synchronisation einer internen Uhr 31 der Bildaufnahmeeinrichtung 30 mit einer internen Uhr 61 der Anregungseinheit 20 hervorrufen sollen, an die interne Uhr 61 über eine Steuereinrichtung 62 des Signalgenerators 60 weiterleiten. Die interne Uhr 61 kann, wie in Figur 1 dargestellt, in dem Signalgenerator 60 angeordnet sein oder eine davon getrennte Komponente sein, die mit dem Signalgenerator 60 zusammenwirkt und dessen Arbeitstakt vorgibt.

Der Signalgenerator 60 umfasst bei dem Ausführungsbeispiel gemäß Figur 1 neben der Uhr 61 und der Steuereinrichtung 62, bei der es sich um eine Recheneinrichtung zum Beispiel in Form eines Mikroprozessors handeln kann, einen Speicher 63, in dem eine Vielzahl an Signalmustern 63a abgespeichert ist. Die Auswahl eines dieser Signalmuster 63a erfolgt durch die Auswerteinrichtung 50 durch Eingabe eines entsprechenden Konfigurationssignals K am Auswahleingang 60b des Signalgenerators 60.

Die Auswerteinrichtung 50 ist vorzugsweise derart ausgestaltet, dass sie im Falle eines am Triggersignalanschluss 20a der Anregungseinheit 20 bzw. am Triggersignaleingang 50e der Auswerteinrichtung 50 anliegenden Signals die Pulslänge und/oder die Pulsamplitude eines ersten Pulses des jeweiligen Signals auswertet und die Feststellung, ob es sich um ein Triggersignal T zur Auslösung eines Schwingungsmusters oder um ein Konfigurationssignal K handelt, anhand der Pulslänge und/oder der Pulsamplitude des ersten Pulses des jeweiligen Signals trifft.

Die Funktionsweise der Elastographieeinrichtung 10 gemäß Figur 1 soll beispielhaft für den Fall näher erläutert werden, dass eine zeitharmonische Elastographie mittels MRT als Bildaufnahmeeinrichtung 30 durchgeführt werden soll:

Die Wiederholzeit der Bildaufnahme soll beispielsweise 100 ms betragen, d.h. das vom Signalgenerator 60 erzeugte Vibrationssignal muss sich exakt alle 100 ms wiederholen, um Interferenzen zwischen Bildaufnahme und Objektbewegung (Gewebematerial) zu vermeiden. Weiterhin soll die Phase der Wellenform innerhalb der 100-ms-Wiederholzeit verschoben werden, um ein kontrolliertes Durchlaufen der Welle im MRT-Bild zu erreichen [3]. Zusätzlich soll nach Aufnahme dieser Phasenschritte der Scherwelle bei einer einzelnen harmonischen Anregungsfrequenz eine weitere Anregungsfrequenz mit passender Periodenzahl und Amplitude im Signalgenerator ausgewählt werden, um die darauf folgende Elastographieuntersuchung mit verändertem zeitharmonischem Stimulus durchzuführen. Erst die kombinierte Auswertung aller zeitharmonischen Wellenfelder ermöglicht die Berechnung besonders hochaufgelöster Karten der viskoelastischen Eigenschaften im Gewebe. Bereits geringe Abweichungen zwischen dem periodischen Verhalten von Vibrationssignal und Bildaufnahme können hingegen zu einer fehlerhaften Abtastung der Gewebeoszillation führen und die Elastographieuntersuchung damit ganz oder in Teilen unbrauchbar machen.

Mit Blick auf diese Problematik wird bei dem Ausführungsbeispiel gemäß Figur 1 eine verzögerungsarme, also instantane bzw. quasi-instantane, Informationsübertragung zwischen der Bildaufnahmeeinrichtung 30 und der Anregungseinheit 20 ermöglicht, welche in der Lage ist,

(i) das Vibrationssignal gewünschter Frequenz, Periodenzahl und Amplitude mittels des Konfigurationssignals K auszuwählen,

(ii) es auszulösen, d.h. im Signalgenerator 60 abzuarbeiten, dabei einen kontrollierten zeitlichen Versatz einzufügen, um unterschiedliche Phasen der Vibration im Gewebe zum Zeitpunkt der Bildaufnahme zu bewirken und

(iii) im weiteren Verlauf der Untersuchung die Re-Synchronisierung des Vibrationssignals mit der Bildaufnahme auszuführen.

In dem nachfolgend beispielhaft näher beschriebenen Verfahren erfolgt die Kommunikation zwischen der Bildaufnahmeeinrichtung 30 und der Anregungseinheit 20 über die Signalleitung 40, nämlich mittels schwellwertbasierter Signale unterschiedlicher Länge und/oder Anzahl zur Übergabe kodierter Informationen. Da diese Signale zusätzliche Information kodieren, werden sie im Folgenden als "Steuerpulse" P bezeichnet. Die Steuerpulse P können unterschiedliche Dauer aufweisen, welche durch die Anregungseinheit 20 in Information zur Auswahl einer Reihe möglicher Aktionen umgewandelt wird. Mögliche Aktionen der Anregungseinheit 20, die von den Steuerpulsen P initiiert werden können, bestehen in Auswahl, Konfiguration, Auslösung, Synchronisierung und Abbruch einzelner Vibrations-Signalformen sowie beliebigen Kombinationen dieser Aktionen.

Zusätzlich kann, im Sinne einer vorteilhaften Erweiterung des Verfahrens, eine umgekehrte Kommunikation von der Anregungseinheit 20 zur Bildaufnahmeeinrichtung 30 über die Signalleitung 40 stattfinden, beispielsweise über Statussignale, wie sie in der Figur 1 mit dem Bezugszeichen STA gekennzeichnet sind. Dieser Kommunikationsweg kann beispielsweise zur Anzeige der Bereitschaft der Anregungseinheit 20 oder dem sicherheitsbedingten Abbruch der Bildaufnahme genutzt werden. Weiterhin kann hierdurch die Anregungseinheit 20 die ordnungsgemäße Abarbeitung eines zuvor gesendeten Befehls bestätigen und somit das Risiko einer Fehlkonfiguration reduzieren.

Das in der Figur 1 gezeigte Ausführungsbeispiel für die Kommunikation zur Konfiguration und Synchronisierung zwischen einem MR-Tomographen und einer Anregungseinheit 20 mittels Steuerpulsen kann beispielsweise in der Mehrfrequenz-MR-Elastographie (MMRE) eingesetzt werden. Beispielsweise können in dem Speicher 63 bis zu einhundert Signalmuster 63a in Form von Protokollen (vorzugsweise bestehend aus einer Vibrations-Signalform, ihrer Dauer, Amplitude und der Anzahl der auszugebenden Zyklen) vorab gespeichert werden. Jedes Protokoll wird vorzugsweise über einen numerischen Index n im Bereich zwischen 0 und 99 identifiziert.

Bei den Signalmustern 63a handelt es sich vorzugsweise um harmonische Signalmuster, also sinusförmige Signalmuster mit einer individuell zugeordneten (einzigen) Schwingfrequenz, einer individuell zugeordneten Amplitude und einer individuell zugeordneten zeitlichen Signalmusterlänge. Die im dem Speicher 63 abgespeicherten Signalmuster 63a unterscheiden sich vorzugsweise mit anderen Worten nur hinsichtlich ihrer (einzigen) Schwingfrequenz, ihrer Amplitude und ihrer Signalmusterlänge.

Während einer Untersuchung wird die Dauer jedes Steuerpulses P von der Auswerteinrichtung 50 ausgewertet: Kurze Pulse mit einer Dauer von 10 Mikrosekunden werden beispielsweise als "reguläre" Triggerpulse T zur Triggerung interpretiert und führen demgemäß zu einer Ausgabe der aktuell konfigurierten Wellenform.

Pulse, deren Dauer beispielsweise 15 Mikrosekunden übersteigen, dienen beispielsweise der Kommunikation zur Konfiguration des Signalgenerators 60. Ihre Dauer dt wird von der Auswerteinrichtung 50 beispielsweise nach der folgenden Formel in einen Protokoll-Index n übersetzt:

$$\mathtt{n = floor((dt - 15~\mu s)/10~\mu s).} \qquad (1)$$

Der Signalgenerator 60 liest in einem solchen Falle die im Protokoll "n" hinterlegte Einstellung, also das jeweils korrespondierende Signalmuster 63a aus dem Speicher 63, aus und konfiguriert die aktive Signalform entsprechend.

Bei dem o. g. vorteilhaften Ausführungsbeispiel führt ein als Konfigurationssignal K erkannter Steuerpuls P somit nicht unmittelbar zur Ausgabe einer Wellenform; erst nach erfolgreicher Konfiguration und nach Eintreffen des nächsten als Triggersignal T erkannten Steuerpulses P kommt es zum Auslösen der jeweils nächsten Vibration.

Auf Seiten der Bildaufnahmeeinrichtung 30, hier also des MRT-Geräts, erfolgt vorzugsweise außerdem eine Anpassung der Messparameter an die jeweils aktuelle Konfiguration des Signalgenerators 60 bzw. das jeweils ausgewählte Signalmuster 63a. Beispielsweise ist es möglich, dass die in der MR-Se-quenz zur Bewegungskodierung verwendete Gradientenform an die Frequenz der von der Anregungseinheit 20 erzeugten Vibration angepasst wird, um maximale Bewegungssensitivität zu erzielen. Durch einen Abgleich der Konfiguration der Bildaufnahmeeinrichtung 30 und der Konfigurationstabelle im Signalgenerator 60 kann damit sichergestellt werden, dass jede Messung mit optimal aufeinander abgestimmten Parametern erfolgt. Hierdurch wird einerseits die Wahrscheinlichkeit einer Fehlmessung durch einen Bedienfehler reduziert; andererseits wird auch der Ablauf einer Untersuchung beschleunigt, weil manuelles Umschalten der Messparameter zwischen den Einzelmessungen entfällt.

Um eine optimale Unempfindlichkeit gegenüber Fluktuationen der Länge der Steuerpulse P zu erreichen, unterscheiden sich vorzugsweise die Algorithmen für die Kodierung und Dekodierung des Index n. Während die Dekodierung durch den Signalgenerator 60 beispielsweise nach o. g. Gleichung (1) erfolgt, wird die Pulsdauer in der Bild-aufnahmeeinrichtung 30 beispielsweise gemäß

$$\mathtt{dt = 20~\mu s + n \bullet 10~\mu s} \qquad (2)$$

umgesetzt. Zu erwähnen sind hier die unterschiedlichen Offsets von 15 und 20 Mikrosekunden in Gleichung (1) bzw. (2). Hierdurch wird eine Toleranz von 5 Mikrosekunden in beide Richtungen (d. h. in Richtung zu langer und zu kurzer Steuerpulse) erreicht. Beispielsweise würde die Einstellung n=1 von der Bildaufnahmeeinrichtung 30 gemäß Gl. (2) als 30 $\mu$s kodiert, während nach Gl. (1) jede Pulsdauer im Bereich 25 $\mu$s $\leq$ dt < 35 $\mu$s zu n=1 dekodiert würde.

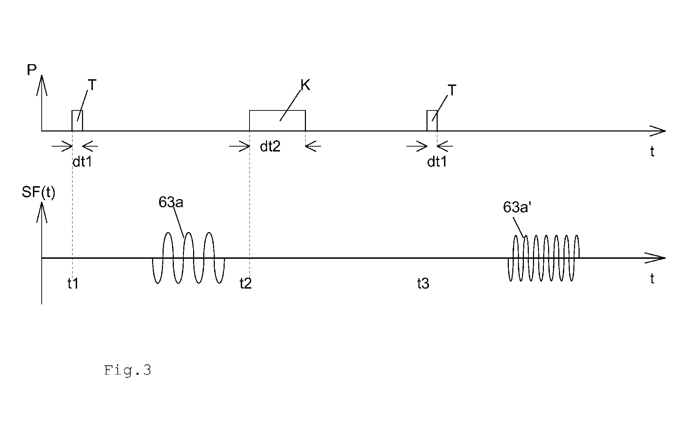

Die Figur 3 zeigt beispielhaft den Zeitverlauf von Steuerpulsen P über der Zeit t, die am Triggersignalanschluss 20a der Anregungseinheit 20 und damit am Triggersignaleingang 50e der Auswerteinrichtung 50 eingehen können, sowie den Zeitverlauf von Signalmustern SF(t), die der Signalgenerator 60 an den elektromechanischen Wandler 70 ausgibt.

In der Figur 3 ist beispielsweise gezeigt, wie zum Zeitpunkt t1 ein Triggersignal T am Triggersignalanschluss 20a der Anregungseinheit ankommt. Die Auswerteinrichtung 50 erkennt in dem Steuerpuls P das Triggersignal T anhand der zeitlichen Länge dt1 des Pulses und leitet das Triggersignal T zum Triggereingang 60a des Signalgenerators 60 weiter. Beispielsweise wird die Auswerteinrichtung 50 in dem Steuerpuls P das Triggersignal T erkennen, wenn die zeitliche Länge dt1 des Pulses maximal 15 $\mu$s beträgt.

Der Signalgenerator 60 erzeugt bei Empfang des Triggersignals T ausgangsseitig ein elektrisches Signalmuster 63a. Das Signalmuster 63a wurde in einem vorherigen Konfigurationsschritt im Signalgenerator 60 eingestellt bzw. aus dem Speicher 63 ausgelesen und aktiviert, indem die harmonische Schwingfrequenz, die Amplitude und die Schwingdauer entsprechend eingestellt wurden.

Zum Zeitpunkt t2 geht bei dem Beispiel gemäß Figur 3 am Triggersignalanschluss 20a der Anregungseinheit 20 ein Steuerpuls P ein, der ein Konfigurationssignal K bildet. Die Auswerteinrichtung 50 erkennt in dem Steuerpuls P das Konfigurationssignal K anhand der zeitlichen Länge dt2 des Pulses und leitet das Konfigurationssignal K zum Auswahleingang 60b des Signalgenerators 60 weiter. Beispielsweise wird die Auswerteinrichtung 50 in dem Steuerpuls P das Konfigurationssignal K erkennen, wenn die zeitliche Pulslänge dt2 minimal 15 $\mu$s beträgt.

Der Signalgenerator 60 stellt bei Empfang des Konfigurationssignals K das Signalmuster auf das neue Signalmuster 63a' um, indem er das neue Signalmuster 63a' aus dem Speicher 63 ausliest und aktiviert und indem er die harmonische Schwingfrequenz, die Amplitude und die Schwingdauer entsprechend neu einstellt.

Zum Zeitpunkt t3 geht bei dem Beispiel gemäß Figur 3 wieder ein Triggersignal T am Triggersignalanschluss 20a der Anregungseinheit 20 ein. Die Auswerteinrichtung 50 erkennt in dem Steuerpuls P das Triggersignal T anhand der zeitlichen Länge dt1 des Pulses und leitet demgemäß das Triggersignal T zum Triggereingang 60a des Signalgenerators 60 weiter. Beispielsweise wird die Auswerteinrichtung 50 in dem Steuerpuls P das Triggersignal T erkennen, wenn die zeitliche Länge dt1 maximal 15 µs beträgt.

Der Signalgenerator 60 erzeugt bei Empfang des Triggersignals T ausgangsseitig das zuvor neu eingestellte Signalmuster 63a'.

Bei dem Ausführungsbeispiel gemäß Figur 3 sind die Steuerpulse P positive Pulse, alternativ können auch negative bzw. invertierte Pulse verwendet werden.

Die bei dem Ausführungsbeispiel gemäß Fig. 3 gezeigte Verzögerung zwischen dem Eingang der Steuerpulse P, insbesondere dem Eingang der Triggersignale T, und der Ausgabe der Signalmuster 63a bzw. 63a' ist nur beispielhaft zu verstehen; die Verzögerung kann variabel sein und von internen Laufzeitverzögerungen in der Auswerteinrichtung 50 und dem Signalgenerator 60 abgesehen beispielsweise auch Null betragen.

Vorteile der oben beschriebenen Elastographieeinrichtung 10 und deren Betriebsverfahren bestehen in

- einer zeitsparenden, automatisierten Echtzeitsteuerung und -konfiguration des Signalgenerators 60 der Elastographieeinrichtung 10,
- der Bereitstellung eines unidirektionalen oder bidirektionalen Kommunikationskanals über eine einzige Signalleitung 40 mit minimaler Latenz zur Übertragung von Triggersignalen T und Konfigurationssignalen K mittels Steuerpulsen P,
- eine kostengünstige Umsetzbarkeit durch Verwendung etablierter Standards und Schnittstellen (beispielhaft:

   TTL-Pulse über Koaxialkabel, oder optische Pulse über Lichtwellenleiter),

- eine einfache nachträgliche Erweiterbarkeit des unterstützten Befehlssatzes durch Modifikation der empfängerseitig hinterlegten Aktionstabelle und Software-Anpassung in der Bildaufnahmeeinrichtung 30 (MR-Tomograph) und
- eine Erweiterbarkeit auf bidirektionale Kommunikation durch Hinzufügen eines zweiten, unabhängigen unidirektionalen Kommunikationskanals.

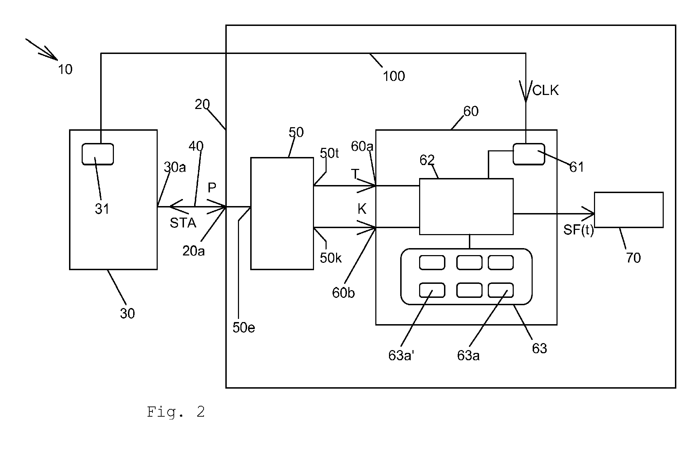

Die Figur 2 zeigt ein weiteres Ausführungsbeispiel für eine Elastographieeinrichtung 10. Die Elastographieeinrichtung 10 entspricht im Wesentlichen der Elastographieeinrichtung 10 gemäß Figur 1 und unterscheidet sich von dieser nur hinsichtlich der zeitlichen Synchronisation der internen Uhr 31 der Bildaufnahmeeinrichtung 30 mit der internen Uhr 61 der Anregungseinheit 20. Zur Synchronisation der beiden Uhren 31 und 61 ist eine separate Synchronisationsleitung 100 vorgesehen, die einen unmittelbaren Austausch von Synchronisationssignalen CLK ermöglicht.

Im übrigen gelten die obigen Erläuterungen zur Figur 1 für das Ausführungsbeispiel gemäß Figur 2 entsprechend.

Bei den Ausführungsbeispielen gemäß den Figuren 1 bis 3 erfolgt die Unterscheidung von Triggersignalen und Konfigurationssignalen sowie die Erkennung von Signalmustern 63a in Konfigurationssignalen allein anhand der Pulslänge der Steuerpulse P; alternativ kann eine Kodierung der Signalmuster 63a auch auf andere Art erfolgen, beispielsweise über die Amplitude der Steuerpulse oder über binär kodierte Steuerpulsfolgen, bei denen beispielsweise jeweils der erste Puls (Startpuls) und/oder weitere Pulse der Steuerpulsfolge die Unterscheidung zwischen Triggersignal und Konfigurationssignal ermöglicht und die weiteren Pulse der jeweiligen Steuerpulsfolgen die gewünschte Konfiguration bestimmen.

Literatur

[1] Venkatesh SK, Yin M, Ehman RL. Magnetic resonance elastography of liver: Technique, analysis, and clinical applications. J Magn Reson Imaging 2013; 37(3): :544-555.

[2] Bamber J, Cosgrove D, Dietrich CF, Fromageau J, Bojunga J, Calliada F, Cantisani V, Correas JM, D'Onofrio M, Drakonaki EE, Fink M, Friedrich-Rust M, Gilja OH, Havre RF, Jenssen C, Klauser AS, Ohlinger R, Saftoiu A, Schaefer F, Sporea I, Piscaglia F. EFSUMB guidelines and recommendations on the clinical use of ultrasound elastography. Part 1: Basic principles and technology. Ultraschall Med 2013;34(2):169-184.

[3] Hirsch S, Guo J, Reiter R, Papazoglou S, Kroencke T, Braun J, Sack I. MR Elastography of the Liver and the Spleen Using a Piezoelectric Driver, Single-Shot Wave-Field Acquisition, and Multifrequency Dual Parameter Reconstruction. Magn Reson Med 2013;71(1):267-277.

Bezugszeichen

| | |
|---|---|
| 10 | Elastographieeinrichtung |
| 20 | Anregungseinheit |
| 20a | Triggersignalanschluss der Anregungseinheit |
| 30 | Bildaufnahmeeinrichtung |
| 30a | Signalausgang der Bildaufnahmeeinrichtung |
| 31 | interne Uhr |
| 40 | Signalleitung |
| 50 | Auswerteinrichtung |
| 50e | Triggersignaleingang |
| 50k | Konfigurationsausgang |
| 50t | Triggersignalausgang |
| 60 | Signalgenerator |
| 60a | Triggereingang des Signalgenerators |
| 60b | Auswahleingang des Signalgenerators |
| 61 | interne Uhr |
| 62 | Steuereinrichtung |
| 63 | Speicher |
| 63a | Signalmuster |
| 63a' | Signalmuster |
| 70 | elektromechanischer Wandler |
| 100 | Synchronisationsleitung |

| | |
|---|---|
| dt | Dauer |
| dt1 | Pulslänge |
| dt2 | Pulslänge |
| CLK | Synchronisationssignale |
| K | Konfigurationssignale |
| P | Steuerpulse |
| SF(t) | Zeitverlauf von Signalmustern |
| STA | Statussignale |
| t | Zeit |
| t1 | Zeitpunkt |
| t2 | Zeitpunkt |
| t3 | Zeitpunkt |
| T | Triggersignale / Triggerpulse |

**Patentansprüche**

1.  Verfahren zum Betreiben einer Elastographieeinrichtung (10), bei dem

    - mit einer Anregungseinheit (20) mechanische Schwingungsmuster zur Erzeugung mechanischer Gewebebewegungen in einem menschlichen oder tierischen Gewebe erzeugt werden und
    - mit einer Bildaufnahmeeinrichtung (30) während der Gewebebewegungen Bilder des Gewebes erzeugt werden,

    **dadurch gekennzeichnet, dass**

    - zur Auslösung des jeweils nächsten mechanischen Schwingungsmusters in einen Triggersignalanschluss (20a) der Anregungseinheit (20) jeweils ein Triggersignal (T) eingespeist wird und
    - zur Konfiguration der Anregungseinheit (20) Konfigurationssignale (K) in denselben Triggersignalanschluss (20a) der Anregungseinheit (20) eingespeist werden.

2.  Verfahren nach Anspruch 1,

**dadurch gekennzeichnet, dass**

- die Anregungseinheit (20) am Triggersignalanschluss (20a) anliegende Signale mit einer Auswerteinrichtung (50) auswertet und Triggersignale (T) zur Auslösung des jeweils nächsten mechanischen Schwingungsmusters von Konfigurationssignalen (K) unterscheidet und
- im Falle des Eingangs eines Konfigurationssignals (K) die in dem Konfigurationssignal (K) definierte Konfigurierung von der Anregungseinheit (20) vorgenommen wird.

3. Verfahren nach einem der voranstehenden Ansprüche,
   **dadurch gekennzeichnet, dass**

   - die Auswerteinrichtung (50) an dem Triggersignalanschluss (20a) anliegende Triggersignale (T) zur Auslösung des jeweils nächsten mechanischen Schwingungsmusters an einen Triggereingang (60a) eines Signalgenerators (60) der Anregungseinheit (20) weiterleitet und
   - die Auswerteinrichtung (50) an dem Triggersignalanschluss (20a) anliegende Konfigurationssignale (K), die jeweils ein in dem Signalgenerator (60) abgespeichertes Signalmuster (63a) aus einer Gruppe von in dem Signalgenerator (60) abgespeicherten Signalmustern (63a) definieren, an einen Auswahleingang (60b) des Signalgenerators (60) zur Auswahl des jeweiligen Signalmusters (63a) weiterleitet.

4. Verfahren nach einem der voranstehenden Ansprüche,
   **dadurch gekennzeichnet, dass**
   die Auswerteinrichtung (50) an dem Triggersignalanschluss (20a) anliegende Konfigurationssignale (K), die jeweils eine zeitliche Synchronisation einer internen Zeitbasis der Bildaufnahmeeinrichtung (30) mit einer internen Zeitbasis (31) der Anregungseinheit (20) hervorrufen sollen, an die interne Zeitbasis (61) der Anregungseinheit (20) weiterleitet.

5. Verfahren nach einem der voranstehenden Ansprüche,
   **dadurch gekennzeichnet, dass**
   die Unterscheidung eines Triggersignals (T) von einem Konfigurationssignal (K) anhand der Pulslänge und/oder der Pulsamplitude und/oder des Pulsmusters des jeweiligen Signals oder zumindest eines ersten Pulses des jeweiligen Signals erfolgt.

6. Verfahren nach einem der voranstehenden Ansprüche,
   **dadurch gekennzeichnet, dass**
   die Auswerteinrichtung (50) zur Übertragung des Gerätestatus der Anregungseinheit (20) Statussignale (STA) an dem Triggersignalanschluss (20a) ausgibt und über diesen Triggersignalanschluss (20a) zu der Bildaufnahmeeinrichtung (30) überträgt.

7. Elastographieeinrichtung (10) mit

   - einer Anregungseinheit (20) zur Erzeugung mechanischer Schwingungsmuster und zur Erzeugung mechanischer Gewebebewegungen in einem menschlichen oder tierischen Gewebe und
   - einer Bildaufnahmeeinrichtung (30),
   **dadurch gekennzeichnet, dass**
   - die Bildaufnahmeeinrichtung (30) einen Signalausgang (30a) aufweist, den sie wahlweise als Triggersignal- oder Konfigurationssignalausgang betreiben kann und über den sie wahlweise Triggersignale (T) zur Auslösung eines mechanischen Schwingungsmusters oder Konfigurationssignale (K) in einen Triggersignalanschluss (20a) einer Anregungseinheit (20) einspeisen kann, und
   - die Anregungseinheit (20) eine mit dem Triggersignalanschluss (20a) in Verbindung stehende Auswerteinrichtung (50) aufweist, die derart ausgestaltet ist, dass sie am Triggersignalanschluss (20a) anliegende Signale auswertet und Triggersignale (T) zur Auslösung eines mechanischen Schwingungsmusters von Konfigurationssignalen (K) unterscheidet.

8. Elastographieeinrichtung (10) nach Anspruch 7,
   **dadurch gekennzeichnet, dass**

   - die Anregungseinheit (20) einen Signalgenerator (60) und einen mit dem Signalgenerator (60) verbundenen elektromechanischen Wandler (70) aufweist, der elektrische Signalmuster des Signalgenerators (60) in korrespondierende mechanische Schwingungsmuster umsetzt,

- ein Triggereingang (60a) des Signalgenerators (60) mit einem Triggersignalausgang (50t) der Auswerteinrichtung (50) in Verbindung steht und die Auswerteinrichtung (50) an dem Triggersignalanschluss (20a) anliegende Triggersignale (T) zur Auslösung eines mechanischen Schwingungsmusters an den Triggereingang (60a) des Signalgenerators (60) weiterleitet, und

- ein Auswahleingang (60b) des Signalgenerators (60) mit einem Konfigurationsausgang (50k) der Auswerteinrichtung (50) in Verbindung steht und die Auswerteinrichtung (50) an dem Triggersignalanschluss (20a) anliegende Konfigurationssignale (K), die jeweils ein ausgewähltes und in dem Signalgenerator (60) abgespeichertes Signalmuster (63a) auswählen, über den Konfigurationsausgang (50k) an den Auswahleingang (60b) des Signalgenerators (60) zur Auswahl des jeweiligen Signalmusters (63a) weiterleitet,

- wobei die Auswerteinrichtung (50) derart ausgestaltet ist, dass sie im Falle eines am Triggersignalanschluss (20a) anliegenden Signals die Pulslänge und/oder die Pulsamplitude des jeweiligen Signals oder die Pulslänge und/oder die Pulsamplitude eines ersten Pulses des jeweiligen Signals auswertet und die Feststellung, ob es sich um ein Triggersignal (T) zur Auslösung eines mechanischen Schwingungsmusters oder um ein Konfigurationssignal (K) handelt, anhand der Pulslänge und/oder der Pulsamplitude trifft.

9. Anregungseinheit (20) für eine Elastographieeinrichtung (10), insbesondere eine solche nach einem der voranstehenden Ansprüche,
**dadurch gekennzeichnet, dass**
die Anregungseinheit (20) eine mit einem Triggersignalanschluss (20a) der Anregungseinheit (20) in Verbindung stehende Auswerteinrichtung (50) aufweist, die derart ausgestaltet ist, dass sie am Triggersignalanschluss (20a) anliegende Signale auswertet und Triggersignale (T) zur Auslösung eines mechanischen Schwingungsmusters von Konfigurationssignalen zur Konfigurierung der Anregungseinheit (20) trennt.

10. Bildaufnahmeeinrichtung (30) für eine Elastographieeinrichtung (10), insbesondere eine solche nach einem der voranstehenden Ansprüche 1-8,
**dadurch gekennzeichnet, dass**
die Bildaufnahmeeinrichtung (30) einen Signalausgang (30a) aufweist, den sie wahlweise als Triggersignal- oder Konfigurationssignalausgang betreiben kann und über den sie wahlweise Triggersignale (T) zur Auslösung eines mechanischen Schwingungsmusters oder Konfigurationssignale (K) in einen Triggersignalanschluss (20a) einer Anregungseinheit (20) einspeisen kann.

Fig. 1

Fig. 2

Fig.3

Europäisches
Patentamt

European
Patent Office

Office européen
des brevets

**EUROPÄISCHER RECHERCHENBERICHT**

Nummer der Anmeldung

EP 15 16 2800

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2010/049029 A1 (LI GENG [CN] ET AL) 25. Februar 2010 (2010-02-25) * Abbildung 11 * * Absätze [0037] - [0040] * | 1-10 | INV. A61B5/00 A61B5/055 G01R33/563 |
| A | US 2011/025333 A1 (EHMAN ERIC C [US] ET AL) 3. Februar 2011 (2011-02-03) * Abbildung 2 * * Absätze [0030], [0040] * | 1-10 | ADD. A61B8/00 |
| A | SEBASTIAN HIRSCH ET AL: "MR Elastography of the Liver and the Spleen Using a Piezoelectric Driver, Single-Shot Wave-Field Acquisition, and Multifrequency Dual Parameter Reconstruction", MAGNETIC RESONANCE IN MEDICINE, Bd. 71, Nr. 1, 14. Februar 2013 (2013-02-14), Seiten 267-277, XP055211788, ISSN: 0740-3194, DOI: 10.1002/mrm.24674 * Absatz "Methods/Imaging Sequence" * | 1-10 | |

RECHERCHIERTE SACHGEBIETE (IPC)

G01R
A61B

Der vorliegende Recherchenbericht wurde für alle Patentansprüche erstellt

| Recherchenort | Abschlußdatum der Recherche | Prüfer |
|---|---|---|
| Den Haag | 8. September 2015 | Albrecht, Ronald |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

...............................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 2800

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

08-09-2015

| Im Recherchenbericht angeführtes Patentdokument | Datum der Veröffentlichung | Mitglied(er) der Patentfamilie | Datum der Veröffentlichung |
|---|---|---|---|
| US 2010049029 A1 | 25-02-2010 | US 2010049029 A1<br>WO 2010020184 A1 | 25-02-2010<br>25-02-2010 |
| US 2011025333 A1 | 03-02-2011 | KEINE | |

EPO FORM P0461

Für nähere Einzelheiten zu diesem Anhang : siehe Amtsblatt des Europäischen Patentamts, Nr.12/82

**EP 2 932 890 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **VENKATESH SK ; YIN M ; EHMAN RL.** Magnetic resonance elastography of liver: Technique, analysis, and clinical applications. *J Magn Reson Imaging,* 2013, vol. 37 (3), 544-555
- **BAMBER J ; COSGROVE D ; DIETRICH CF ; FROMAGEAU J ; BOJUNGA J ; CALLIADA F ; CANTISANI V ; CORREAS JM ; D'ONOFRIO M ; DRAKONAKI EE.** EFSUMB guidelines and recommendations on the clinical use of ultrasound elastography. Part 1: Basic principles and technology. *Ultraschall Med,* 2013, vol. 34 (2), 169-184
- **HIRSCH S ; GUO J ; REITER R ; PAPAZOGLOU S ; KROENCKE T ; BRAUN J ; SACK I.** MR Elastography of the Liver and the Spleen Using a Piezoelectric Driver, Single-Shot Wave-Field Acquisition, and Multifrequency Dual Parameter Reconstruction. *Magn Reson Med,* 2013, vol. 71 (1), 267-277